# EUROPEAN PATENT APPLICATION

(11) **EP 1 426 025 A1**
(43) Date of publication of application: **09.06.2004**
(21) Application number: 02771755.2
(22) Date of filing: 21.05.2002
(51) Int. Cl.: A61F 13/15

(54) **INTERLABIAL PAD AND PACKAGE THEREOF**

(30) Priority: 22.05.2001 JP 2001152403; 12.09.2001 JP 2001276338
(71) Applicant: UNI-CHARM CORPORATION, Kawanoe-shi Ehime 799-0111 (JP)
(72) Inventor: MIZUTANI, Satoshi, Technical Center Uni-Charm Corp, Mitoyo-gun, Kagawa 769-1602 (JP); YAMAKI, Koichi, Technical Center, Uni-Charm Corp, Mitoyo-gun, Kagawa 769-1602 (JP); NODA, Yuki, Technical Center, Uni-Charm Corp, Mitoyo-gun, Kagawa 769-1602 (JP); TOKUMOTO, Megumi, Technical Center, Uni-Charm Corp, Mitoyo-gun, Kagawa 769-1602 (JP); SAKAI, Akane, Technical Center, Uni-Charm Corp, Mitoyo-gun, Kagawa 769-1602 (JP)
(74) Representative: Purdy, Hugh Barry
(86) International application number: PCT/JP2002/004895
(87) International publication number: WO 2002/094159

(57) **Abstract**

The present invention provides an interlabial pad, which includes an absorber (13) and comprises an absorbing sheet portion (14a), which faces against the body side in wearing the pad and a support sheet portion (14b), which backs the absorber (14a). The absorber (14a) comprises of a pair of absorbing sheet bodies (16) which are divided along the substantial center line of the interlabial pad 14, the absorbing sheet (16) and the impermeable support sheet (12) are bonded with each other on the peripheral edge portion (15) of the interlabial pad (14) to form a void portion (18) between the absorbing sheet body (16) and the support sheet (12) through which the menstrual blood can flow into. Thereby the menstrual blood flowing down along the inner wall of the labia can be rapidly transferred into the absorber and the shape fitness (fitting ability) of the pad in labia can be improved.

## Description

### Background of the Invention

### Technical Field

The present invention relates to an interlabial pad, which is used for attaching within female labia, particularly relates to the interlabial pad, which can be used together with a sanitary napkin as well as relates to a wrapping body that the interlabial pad is contained in a package for wrapping each of the interlabial pad.

### Background Art

Conventionally, a sanitary napkin and a tampon are used generally as sanitary products for female. However, there has been problem that, as for the sanitary napkin, it is used by bringing into contact with garment, and it tends to cause the leak of menstrual blood from the gap caused by poor adhesion near the ostium vaginae. While there has been another problem that, as for the tampon, on the basis of the nature of its products, it tends to cause the foreign feeling and the discomfort, when wearing it, and it is difficult to fix into the vagina.

Under such situation, sanitary products of the interlabial pad have attracted people in recent years as a sanitary product positioned between the sanitary napkin and the tampon.

The interlabial pad is used by inserting its portion between the labia and bringing into contact with the labia. The interlabial pad has advantages that it excels in a wear feeling and its comfort because of being small as compared with the sanitary napkin and that it is sanitary and clean because the range of the body soiled with menstrual blood is narrow. Moreover, it has characteristics that it is difficult to cause the leak of menstrual blood because of higher adhesion to the body than that of the sanitary napkin and that psychological resistance on wearing is lower than that of the tampon which is inserted into the vagina.

However, a conventional interlabial pad has had a problem that the menstrual blood flows and leaks out of the pad surface before it is absorbed by the absorber. Since the menstrual blood flows along the inner wall of labia at a slow rate with a high degree of wetting to the inner wall of the labia, the menstrual blood does not flow out of the labia gradually but also a lot of the menstrual blood flows out all at once after retention in the labia.

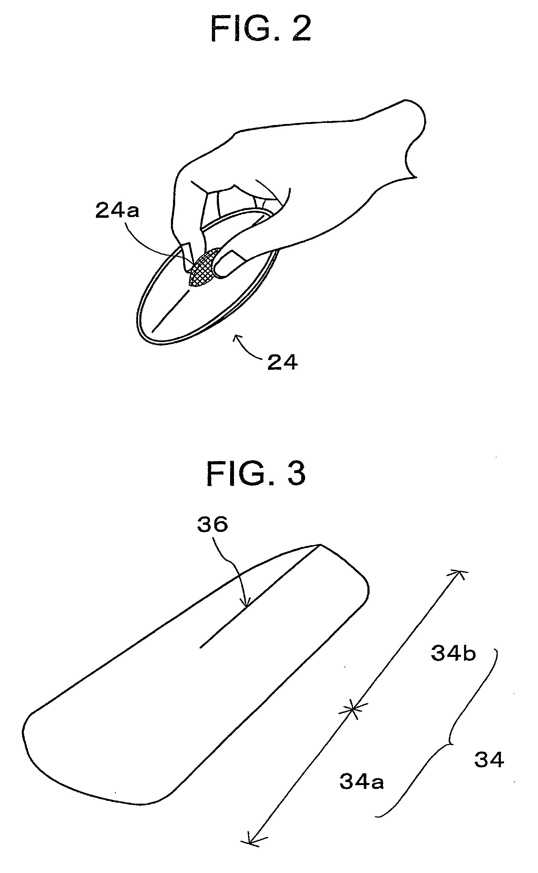

For example, as illustrated in Fig. 2, the interlabial pad 24 has a knob 24a at the side of the garment, thereby a person can wear it easily by holding the knob 24a by fingers (the publication of WO99/56689). However, the pad disclosed by the document lacks adhesion to the inner wall of the labia, since, except for the neighborhood of the knob, it is difficult to apply press force for pushing the pad into the labia. Therefore, before the absorber absorbs the menstrual blood, the menstrual blood flows out of the pad surface, and the leak of the menstrual blood has been occurred.

Further as illustrated in Fig. 3, the interlabial pad 34 is designed that the convex portion can be formed by folding a rear side area 34b along the substantial center line 36 in the longitudinal direction of the pad. As compared with the interlabial pad 24 illustrated in Fig. 2, this interlabial pad 34 can be used by putting the convex portion between labia and improves in the adhesion to the interlabial inner wall. Although the pad has the structure as described hereinbefore, the menstrual blood flows easily out of the pad surface and there has been likelihood of the leak of menstrual blood. Therefore, due to a smaller size of the interlabial pad than that of a sanitary napkin, the serious damage from the leak of menstrual blood has been caused.

### Summary of the Invention

In respect of problems described hereinbefore, the object of the present invention provides an interlabial pad in capable of rapidly carrying the menstrual blood, which flows along the inner wall of the interlabial to the absorber.

To solve the aforementioned problems, the present invention provides an absorbing sheet portion comprising a pair of absorbing sheet bodies which are divided along a substantial center line of the interlabial pad, the absorbing sheet body and the impermeable support sheet are bonded on a peripheral edge portion of the interlabial pad to form a void through which the menstrual blood can flow between the absorbing sheet body and the support sheet, thereby the menstrual blood which flows along an inner wall of labia is rapidly transferred into the absorber, and the shape fitness (fitting ability) in labia can increase.

Concretely, the present invention provides the interlabial pad as follows;

(1) An interlabial pad for attaching to labia, comprising: an absorber for absorbing a body fluid; an absorbing sheet portion facing to a body side upon wearing the interlabial pad; and a support sheet portion backing the absorbing sheet portion; wherein said absorbing sheet portion comprises a pair of absorbing sheet bodies which are separated along a substantial center line of the interlabial pad, and said absorbing sheet body is bonded to said support sheet portion comprising an impermeable support sheet on a peripheral edge portion of the interlabial pad, thereby the body fluid can flow into a void which is formed between a garment face side of the absorbing sheet body and the body face side of the support sheet.

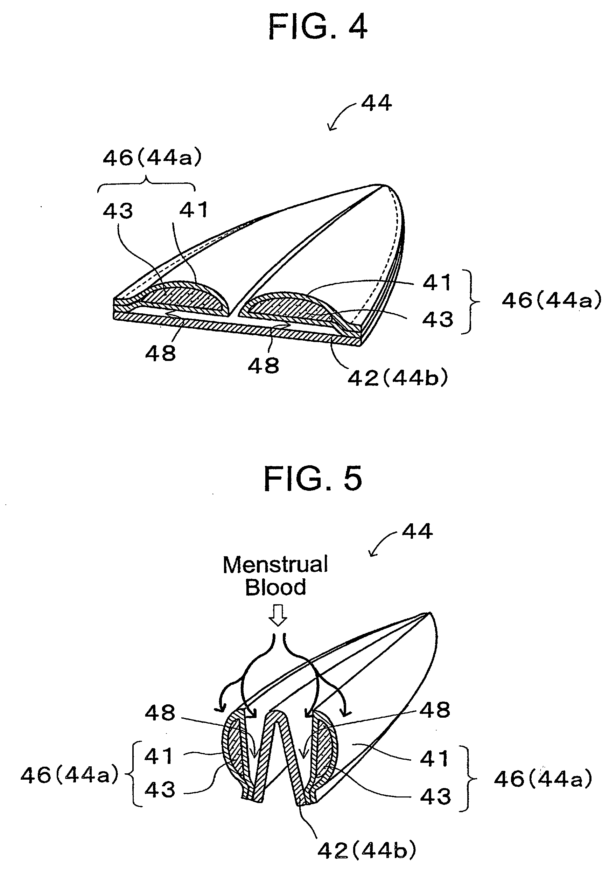

An interlabial pad of the present invention, for example, as shown in Fig.4, comprises an absorbing sheet portion 44a and a support sheet portion 44b backing the absorbing sheet 44a. The absorbing sheet 44a is used to face against the body side upon wearing. The absorbing sheet 44a includes an absorber 43 for absorbing the body fluid, for example, the menstrual blood, and a surface thereof is applied by a water permeable cover sheet 41 if desired. The absorbing sheet 44a comprises a pair of absorbing sheet bodies 46 which is divided along a substantial center line of the interlabial pad 44. While the support sheet portion 44b comprises an impermeable support sheet 42.

The absorbing sheet body 46 and the support sheet 42 are bonded with each other on the peripheral edge portion of the interlabial pad 44, and contacting surfaces other than the peripheral edge portion are not bonded. Therefore, a void portion 48 forms between the garment face side of the absorbing sheet body 46 and the body face side of the support sheet 42 to receive the menstrual blood therethrough.

In the embodiment described hereinbefore, for example, as shown in Fig. 5, the interlabial pad 44 is folded along the substantial center line (that is a dividing line of the absorber) and inserted into the labia, each absorbing sheet body 46 adheres to the inner wall of the labia, while a pocket-like void portion 48 is formed between the garment face side of the absorbing sheet body 46 and the body face side of the support sheet 42. Therefore, the discharged menstrual blood rapidly flows into the void 48 to be absorbed into the garment face side (contacting surface with the support sheet 42) of the absorbing sheet body 46 and to be also absorbed into the body face side (the contacting surface with the inner wall of the labia) of the absorbing sheet 46. Therefore, in comparison of the example that the absorbing sheet body 46 and the support sheet 42 are completely connected with each other at the contacting surface, an increased surface area of the absorber 43 improves absorption efficiency and the menstrual blood can be rapidly transferred into the absorber 43.

Further the absorbing sheet portion 44a comprises a pair of absorbing sheet bodies 46, and is divided along the substantial center line of the pad 44, thereby in comparison with a pad comprised of the absorbing sheet body 46 integrally, the pad of the present invention is easily folded to improve the property of following shapes, and the shape fitness in labia is improved.

(2) The interlabial pad according to (1), wherein a side end of the interlabial pad has a shape of an arc, and both peripheral ends of the arc converge toward edge ends of the substantial center line along a longitudinal direction of the interlabial pad.

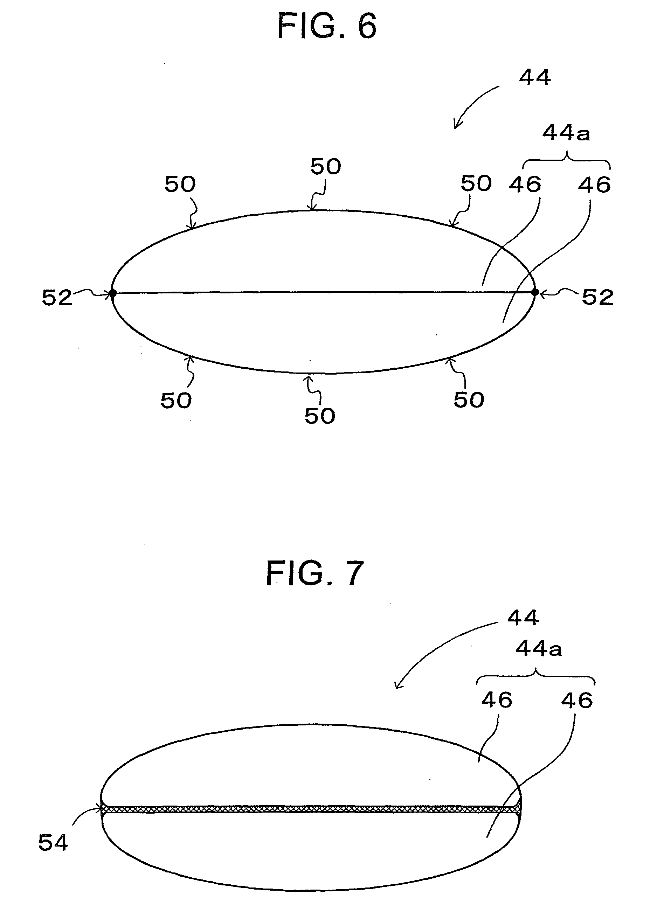

An overall configuration of the interlabial pad of the present invention is not limited as long as the configuration is suitable to attach the pad to labia. For example, an elliptic shape, an ovoid shape, a gourd shape or a drop shape and the like will be eligible. However, in consideration of the shape fitness with labia, for example, as illustrated in Fig. 6, it prefers that the side end 50 of the pad 44 has an arc shape and both peripheral ends of the arc shape converge toward the peripheral end 52 of the substantial center line in a longitudinal direction of the pad 44.

(3) The interlabial pad according to (1) or (2), wherein said pair of absorbing sheet bodies are separated each other, and a groove portion is provided between the absorbing sheet bodies.

In the interlabial pad of the present invention, it is not limited to the embodiment that a pair of absorbing sheet bodies are arranged in neighbor each other, and may provide a groove portion between a pair of absorbing sheet bodies. For example, as illustrated in Fig. 7, the groove portion 54 can be provided between absorbing sheet bodies 46 such that a pair of absorbing sheet bodies 46 are separated from each other with an interval of 10mm.

In a normal interlabial pad, once the menstrual blood transmits the cover sheet once, a part of the menstrual blood solidifies and remains in the cover sheet. Even though the absorber still has an absorption capacity of the menstrual blood, it has tendency not to absorb the menstrual blood any more. In case of providing the groove portion 54 as described hereinbefore, the menstrual blood moves rapidly along the groove portion 54 in forward and backward directions. Therefore, the absorber 43 contained in the absorbing sheet body 46 can be used effectively and can demonstrate its absorption capacity.

(4) The interlabial pad according to any one of (1) to (3), wherein on a body face side of the support sheet, an strip-shaped absorber is disposed along a dividing line of a pair of absorbing sheet bodies.

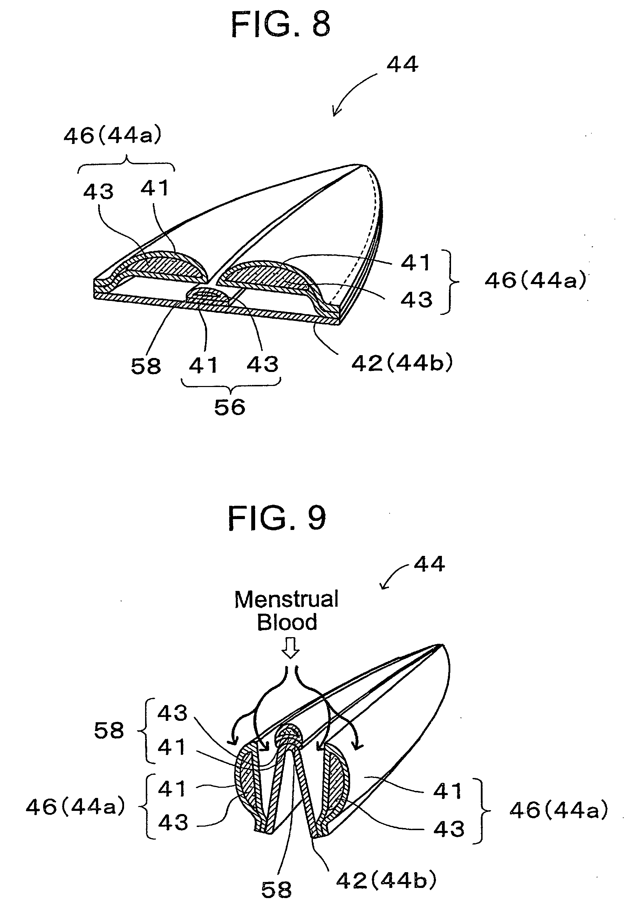

The interlabial pad of the present invention may be structured, for example, as shown in Fig. 8, such that the strip-shaped absorber 58 is provided on the body face side of the support sheet 42 along the dividing line of a pair of absorbing sheet bodies 46 (along the substantial center line of the longitudinal direction of the interlabial pad 44).

As illustrated in Fig. 9, the interlabial pad 44 is folded along the dividing line of a pair of absorbing sheet body 46 and a strip-shaped absorber 56 appears, and the pad is inserted into the labia under the conditions, the strip-shaped absorber 58 contacts closely with the depth portion of labia and a pair of absorbing sheet bodies 46 contact with the inner wall of labia closely. Therefore, almost all of menstrual blood discharged from ostium vaginae is absorbed primarily by the strip-shaped absorber 56 which contacts closely with the depth portion of labia including neighborhood of ostium vaginae, even if a lot of the menstrual blood flow out at once, the menstrual blood is absorbed secondarily by a pair of absorbing sheet bodies 46 which contacts with the inner wall of labia. As described hereinbefore, a lot of menstrual blood, which flows down at once along the labia inner wall, can be transferred rapidly to the absorber 43 by a combination of the strip-shaped absorber 56 and a pair of absorbing sheet bodies 46.

(5) The interlabial pad according to any one of (1) to (4), further including a sheet-shaped absorber being disposed on a body face side of the support sheet, wherein a void is formed between the garment face side of the absorbing sheet body and the body face side of the sheet-shaped absorber whereby body fluid can flow into said void.

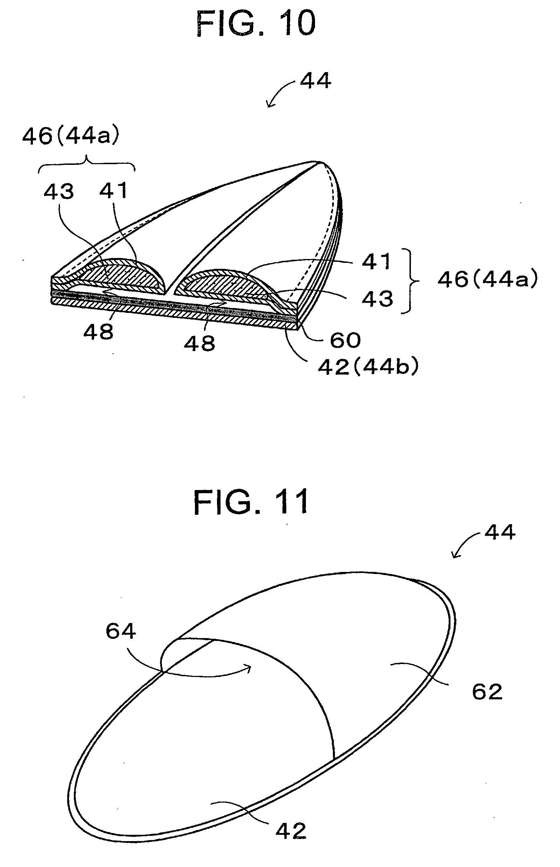

In the interlabial pad of the present invention, for example, as shown in Fig.10, it prefers to dispose the sheet-shaped absorber 60 between the garment face side of the absorbing sheet bodies (more concretely the garment face side of the cover sheet 41) and the body face side of the support sheet 42. The structure described hereinbefore allows the menstrual blood to flow into the void portion 48 between the garment face side of the absorbing sheet bodies 46 and the body face side of the sheet-shaped absorber 60, and the flown blood is absorbed by not only the absorber 43 contained in the absorbing sheet body 46 but also by the sheet shaped absorber 60. Therefore, the menstrual blood can be rapidly transferred into the absorber43 and a lot of menstrual blood at high rate can be absorbed by the pad.

In this embodiment, it is more preferable that the fiber contained in the sheet-shaped absorber 60 is oriented in a lateral direction of the interlabial pad 44, since the absorbed menstrual blood can easily transfer to the oriented direction of the fiber, that is, in left and right sides of the body, and the absorber can be used effectively.

(6) The interlabial pad according to any one of (1) to (5), further comprising a mini sheet piece in the garment face side of the support sheet, including: one or more bonding portions on each side portion in respect of a longitudinal direction of the support sheet; and one or more disconnecting portion in a lateral direction of the support sheet, wherein at least one of said one or more disconnecting portion form an opening for receiving a finger between the mini sheet piece and the support sheet so as to directly keep the opening having a finger breadth in a surface direction of the support sheet.

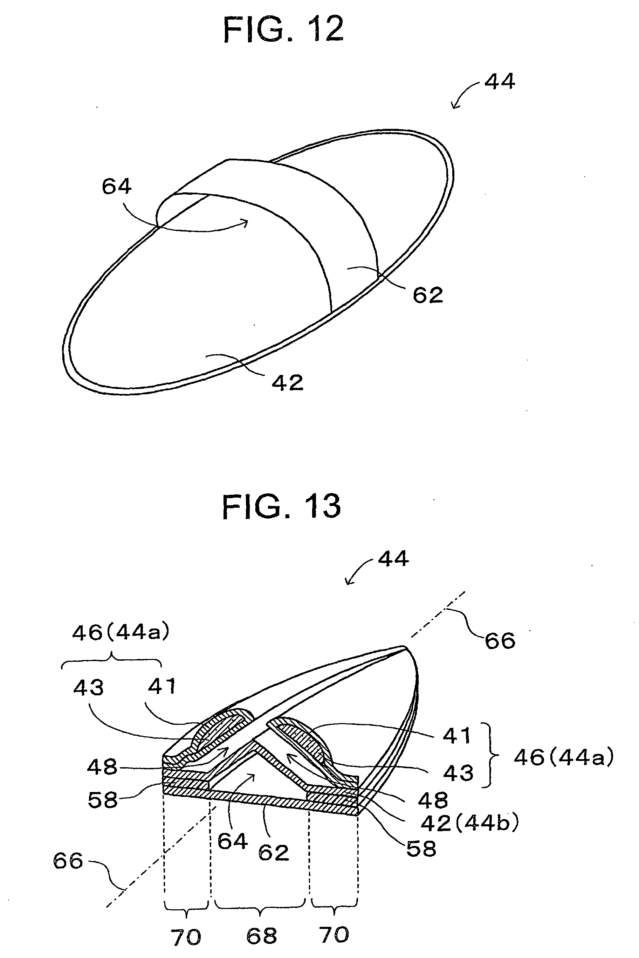

In the interlabial pad of the present invention, for example, as illustrated in Figs. 11 or 12, a mini sheet piece 62 is disposed to form an opening 64 for receiving a finger. In Figs. 11 and 12, in a lateral direction of the support sheet 42, at least one of both sleeve portions of the mini sheet piece 62 is not bonded with the surface of the support sheet 42. Thereby the opening is formed between one sleeve side of the mini sheet piece 52, which is in a non-bonding condition, and the support sheet 42 to form the opening 64 for inserting a finger which is capable of inserting the finger.

In a longitudinal direction of the support sheet 42, the mini sheet piece 62 is bonded with only both lateral sides of the support sheet 42 and is not bonded (adhered) with the inside thereof. Therefore, the mini sheet piece 62 is provided from one lateral side of the support sheet 42 to the other lateral side thereof to bridge both of lateral sides; therefore, at the portion where the bridge is formed from one lateral side to the other, penetrating or non-penetrating space (space for inserting the finger) is formed. A finger can insert such space and hold the pad.

The word "finger breadth" in this specification does not mean the thickness of the finger but the width of the finger in the spread direction of the nail concretely. The opening of the finger breadth means an opening having a sufficient size to insert the finger.

Further, the opening of the finger breadth is "directly kept" in a direction of the support sheet surface. This means that when the finger is inserted into the pad naturally wear the pad, (the ball of a finger is directed to the garment face side of the support sheet and is inserted to maintain the condition), the pad of itself is formed primarily to be adequate for inserting the finger. Therefore, the following case is excluded from the aforementioned example, such that the opening for the finger breadth is kept in a surface direction by rotating the finger after the person inserts the finger, and the opening for the finger breadth is formed on the support sheet surface side secondarily.

As described hereinbefore, in the pad provided with the mini sheet piece, the finger is inserted into the finger insertion opening, thereby the pad can be kept and fixed at the finger temporarily. In this case, the opening for inserting a finger is formed to be the opening for the finger breadth of a wearer, and the flat-shaped finger tip is prevented from directing to different directions in respect of the support sheet, and is inserted to contact with the support sheet surface naturally. That is, the opening for inserting the finger has a wide shape in directions of the support sheet surface in accordance with the shape of the finger tip of the person to wear, and therefore, the wearer is intended to act that the direction where the finger is inserted is determined to detect a fix point of the pad by the ball of the finger tip. Thereby, in attaching the pad between labia where the wearer is difficult to confirm by eyes, the pad can be attached at the adequate position by correctly holding a correct attachment point.

Incidentally, in the present invention, not only the peripheral edge portion of the pad but also the neighborhood to the peripheral edge portion to where the mini sheet piece can be bonded are included in the "side portion" in the longitudinal direction of the support sheet.

(7) The interlabial pad according to (6), wherein at least a pair of absorbing sheet bodies include: an inclining portion having a mountain shape in a body side direction, the mountain shape having a vertex in the substantial center line along the longitudinal direction of the interlabial pad and a pair of side ends; and a hem portion continuing from both of the side ends of said inclining portion, wherein the mini sheet piece is bonded on the garment side of said hem portion.

In the interlabial pad of the present invention, for example, as illustrated in Fig. 13, preferably a pair of the absorbing sheet bodies 46 (that is, the cover sheet 41 and the absorber 43) comprises an inclining portion 68 and a hem portion 70. The inclining portion 68 has a mountain shape toward the body side direction along the substantial center line 66 of the longitudinal direction of the interlabial pad 44. The hem portion 70 continues from both side ends of the inclining portion 68. The mini sheet piece 62 is bonded with the garment side of the hem portion 70.

The aforementioned structure allows the absorbing sheet body 46 to easily progress into the depth of the labia, and a whole shape of the interlabial pad 44 has a form easily capable of effecting a pinching force of labia. Therefore, the pad can provide the user a good adhesion and a wear feeling. Further the finger is inserted into the finger insertion opening 64 to belong the ball of the finger with the central fold line of the inclining portion 68, and the interlabial pad 44 is maintained, thereby the interlabial pad 44 can be guided correctly toward the labia which is difficult to confirm by eyes and can be easily attached thereto.

Incidentally, in this embodiment, it is sufficient to comprise at least the absorbing sheet portion (that is, the cover sheet 41 and the absorber 43) in a mountain shape. As illustrated in Fig. 13, the embodiment, which the support sheet 42 has a mountain shape, is of course included in a scope of present the invention.

(8) The interlabial pad according to any one of (1) to (7), wherein the interlabial pad is used together with a sanitary napkin.

There have been users to use a plurality of sanitary napkins (as referred a "napkin" in following description) laminated with each other when a large volume of menstrual blood flows. However, there has been a problem of bad wear feeling such as stiffness, and it is noticeable from the outside of the garment to the presence of the pad. Further, laminated napkins cover portions other than the neighborhood of ostium vaginae where the laminated use is unnecessary, thereby causing a rash or a sweat. However, when the pad and the napkin are used together, the sanitary products are laminated only around the ostium vaginae, thereby the aforementioned problem can be solved. Further only the pad can be changed without changing the napkin, thereby the wearer does not have to carry a napkin, which has a relatively large size. The sanitary napkin may include not only the napkin in the market for absorbing the menstrual blood but also the sheet for absorbing the vaginal discharge.

(9) The interlabial pad according to any one of (1) to (8), wherein the interlabial pad is a pad for incontinence of urine.

According to the interlabial pad of the present invention, the pad can be used for a pad for absorbing incontinence of urine. That is, ostium vaginae where the menstrual blood is discharged and the urethral meatus, where urine is discharged locate between labia, and the interlabial pad of the present invention to be used between labia can absorb urine also.

As described hereinbefore, the pad of the present invention can absorb urine between labia, especially around the urethral opening and is useful for the absorbing pad for incontinence of urine, especially for a light incontinence of urine.

(10) The interlabial pad according to any one of (1) to (8), wherein the interlabial pad is a pad for absorbing vaginal discharge.

In accordance with the invention, the interlabial pad can be used for the pad of absorbing the vaginal discharge. That is, the interlabial pad is used between labia and can absorb the excretion (vaginal discharge) other than the menstrual blood from ostium vaginae. Therefore, the interlabial pad may be used for the application (for absorbing the vaginal discharge).

As described above, the pad can absorb the vaginal discharge in order to decrease the discomfort for the wearer, and is useful for the wearer who is not menstruating.

(11) The wrapping body comprising: the interlabial pad according to (1) to (10); and a wrapping container for wrapping the interlabial pad individually.

When the interlabial pad is packaged individually, the pad can be carried one by one (each individual packages). As compared with a plurality of pads contained in one package, the pad is kept sanitarily, can be easily carried and can be treated simply.

(12). The wrapping body comprising: the interlabial pad according to any one of (6) to (10); and a wrapping container for wrapping the interlabial pad individually, said wrapping container having an unwrapped opening, wherein said interlabial pad is so contained in said wrapping container that said finger insertion opening faces and opens toward the unwrapped opening of the wrapping container.

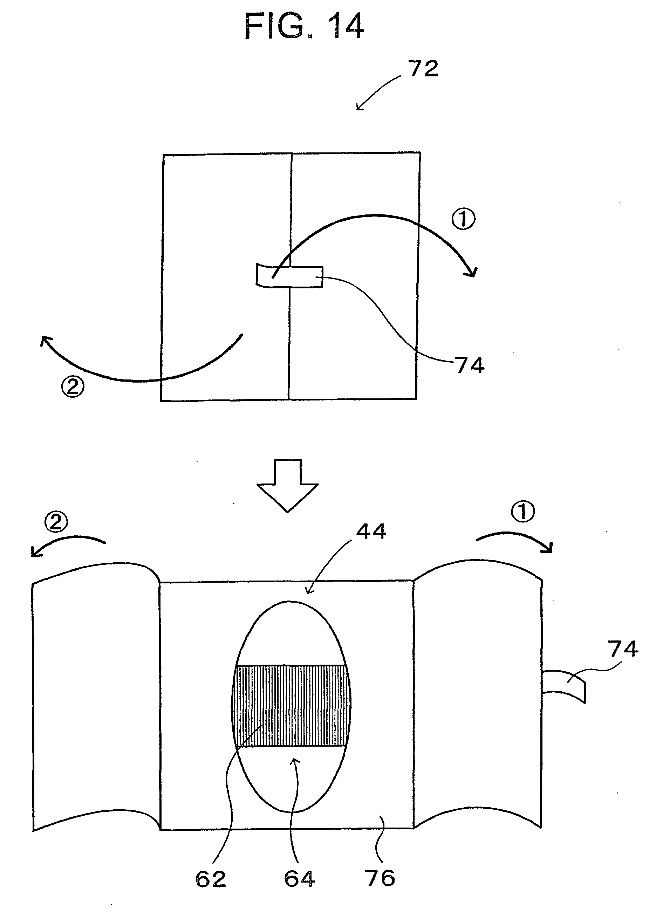

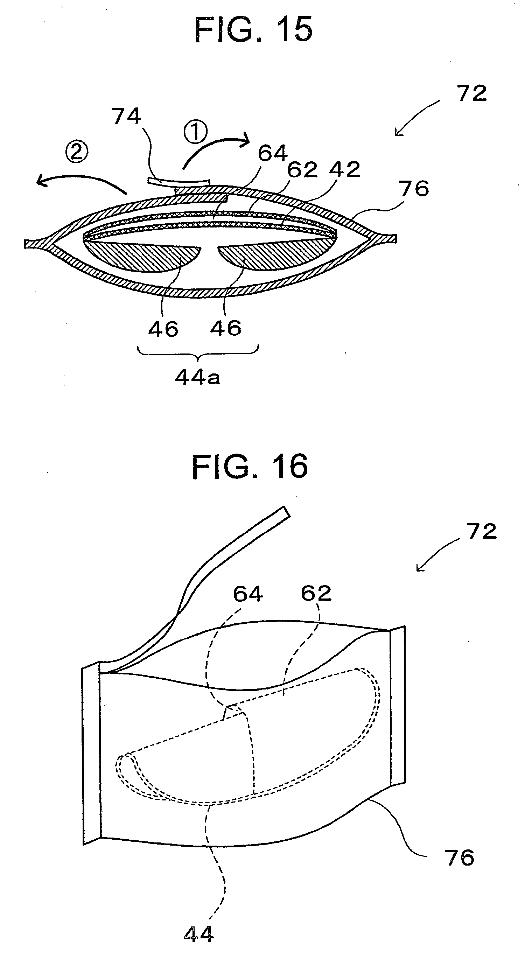

As illustrated in Fig. 14, "said finger insertion opening faces and opens toward the unwrapped opening" illustrates that, upon the wrapping body 72 being opened, the mini sheet piece 62 and further the finger insertion opening 64, which is formed by the piece, appear and, immediately, the finger can be inserted into the finger insertion opening 64. For example, as illustrated in Figs. 14 and Fig. 15, the wrapping body 72 can be opened by pulling a tub tape 74, which is provided at the upper surface side of the wrapping container 76, toward the right side of the drawing. Finger insertion opening 64 appears at the opening of the wrapping body to open toward the unwrapped opening. Therefore the user can insert the finger immediately into the finger insertion opening 64.

(13) The wrapping body according to (12), wherein the interlabial pad is contained in the wrapping container such that the mini sheet piece is mountain-folded toward garment side directions along the substantial center line in the longitudinal direction of the interlabial pad.

"To be mountain-folded toward garment side directions," means that the sheet is completely folded in forming the garment side to be a convex, and further includes the case that, as illustrated in Fig. 16, the sheet is inflected in forming the garment side to be a convex. As described hereinbefore, the interlabial pad 44 is contained in the wrapping container 76, thereby the folded finger insertion opening 64 is naturally opened at the time of breaking the seal: Therefore, the wearer can confirm easily the part where she inserts her finger so as to achieve more smooth and simple attachment of the pad.

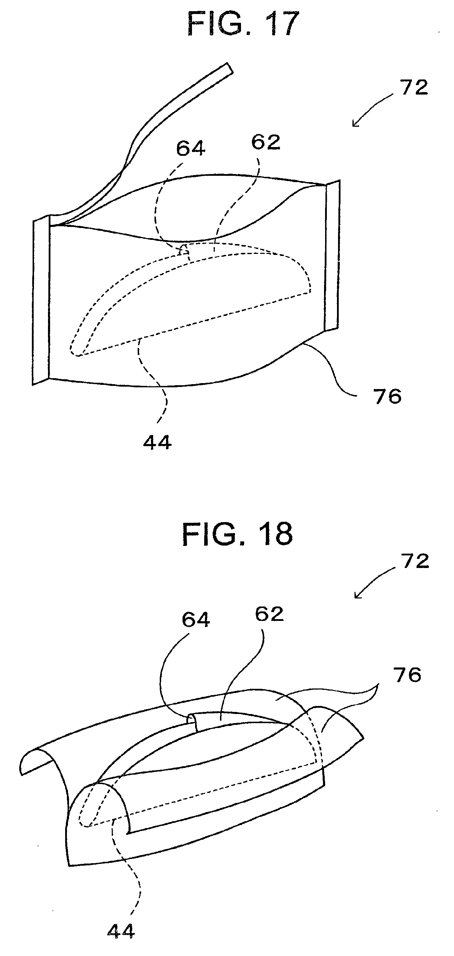

In this embodiment, the mini sheet piece may be a mountain-folded shape and it is not required that a whole pad is in a mountain-folded shape. Therefore, for example, as illustrated in Fig. 16, in the wrapping body 72, a whole interlabial pad 44 is folded in a mountain-folded shape and contained in the wrapping container 76. Further as illustrated in Fig. 17, in the wrapping body 72, only the mini sheet piece 62 is folded in the mountain-folded shape, while the main body of the interlabial pad 44 is folded in a V-shaped toward the garment side and contained in the wrapping container 76.

Further, the method of breaking the seal of the wrapping container is not limited specifically. As illustrated in Figs. 16 and Fig. 17, the wrapping container 76 of which an upper end side is cut to break it is eligible. Further as illustrated in Fig. 18, a wrapping container 76 which is broken the seal from the upper end to both sides (what is called a set of folding doors) is eligible.

### Brief Description of the Drawings

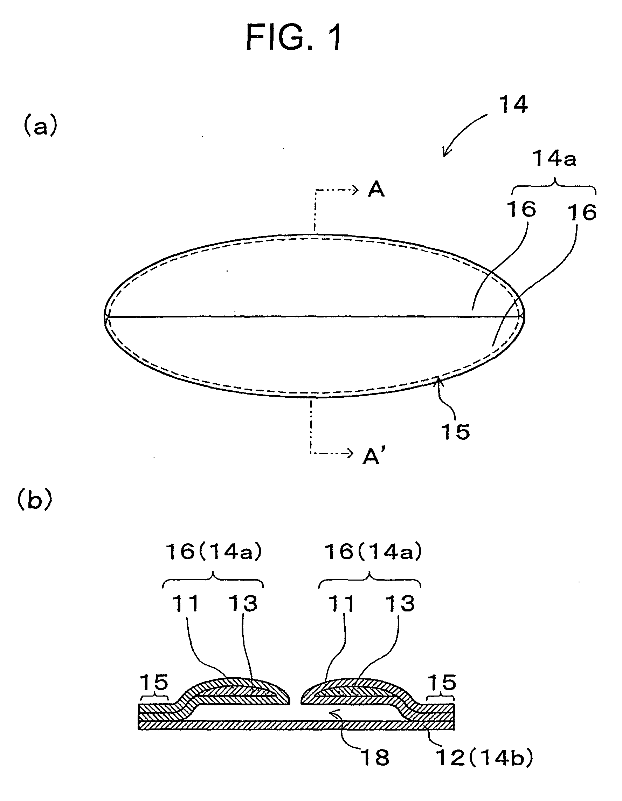

Fig. 1(a) is a cross sectional view along line A-A' of Fig. 1(b) of a structure of an interlabial pad according to the invention. Fig. 1(b) is a top view of the interlabial pad.
Fig. 2 is a perspective view of a structure of a conventional interlabial pad.
Fig. 3 is a perspective view of a structure of a conventional interlabial pad.
Fig. 4 is a perspective view of a cross section of the interlabial pad according to the invention.
Fig. 5 is a perspective view of a cross section of the interlabial pad according to the invention.
Fig. 6 is a top view of the structure of the interlabial pad according to the invention.
Fig. 7 is a top view of the structure of the interlabial pad according to the invention.
Fig. 8 is a perspective view of a cross section of the interlabial pad according to the invention.
Fig. 9 is a perspective view of a cross section of the interlabial pad according to the invention.
Fig. 10 is a perspective view of a cross section of the interlabial pad according to the invention.
Fig. 11 is a perspective view of the structure of the interlabial pad according to the invention.
Fig. 12 is a perspective view of a structure of the interlabial pad according to the invention.
Fig. 13 is a perspective view of a cross section of the interlabial pad according to the invention.
Fig. 14 is a process diagram of a way of breaking a seal of a wrapping body according to the invention.
Fig. 15 is a cross section view of a structure of the wrapping body according to the invention.
Fig. 16 is a perspective view of a structure of the wrapping body according to the invention.
Fig. 17 is a perspective view of a structure of the wrapping body according to the invention.
Fig. 18 is a perspective view of a structure of the wrapping body according to the invention.

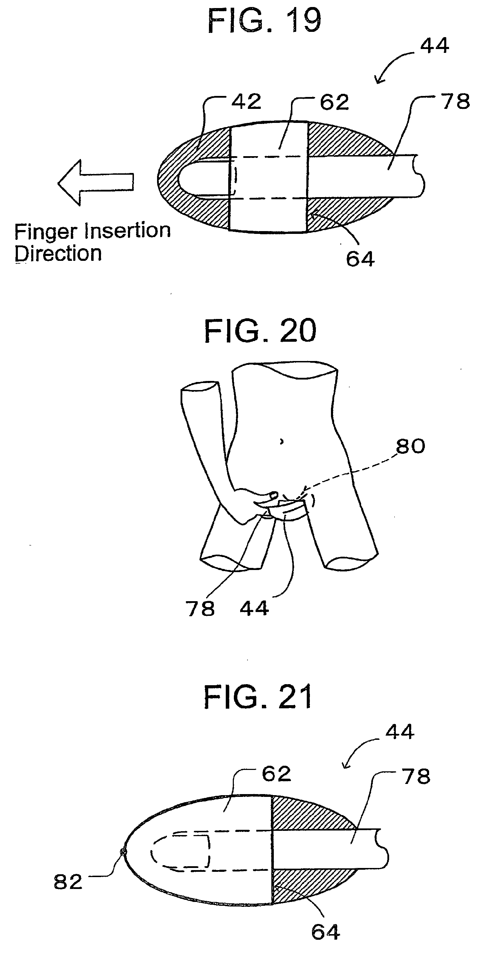

Fig. 19 is a top view of an embodiment of a mini sheet piece of the interlabial pad according to the invention.
Fig. 20 is an explanation view of a way of wearing the interlabial pad according to the invention.
Fig. 21 is a top view of an embodiment of a mini sheet piece of the interlabial pad according to the invention.

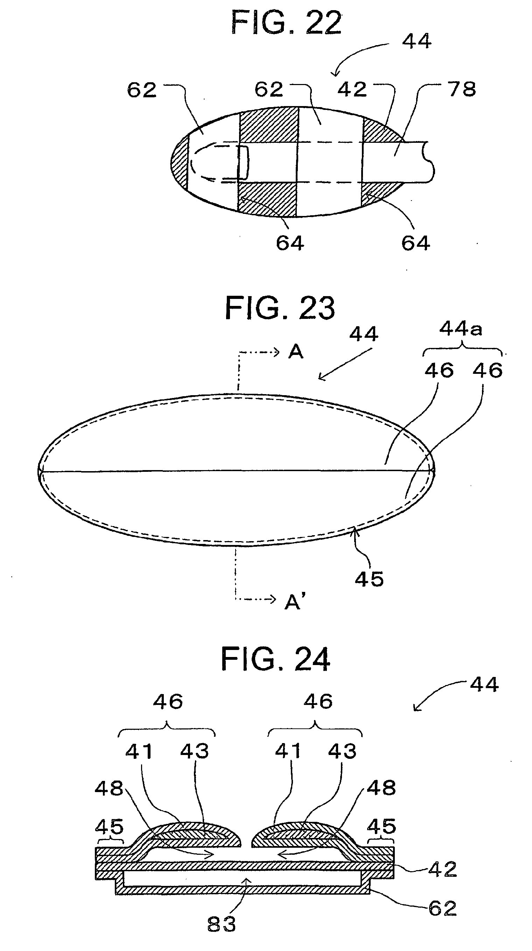

Fig. 22 is a top view of an embodiment of the interlabial pad according to the invention.
Fig. 23 is a top view of the structure of the interlabial pad according to the invention.
Fig. 24 is a cross section view along line A-A' of the interlabial pad of Fig. 22.

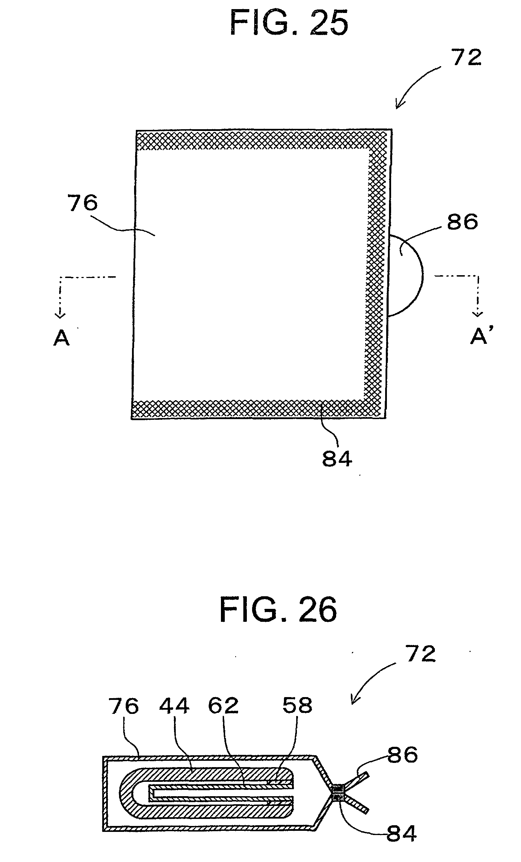

Fig. 25 is a top view of one embodiment of the wrapping body according to the invention.
Fig. 26 is a cross section view along line A-A' of the wrapping body of Fig. 25.

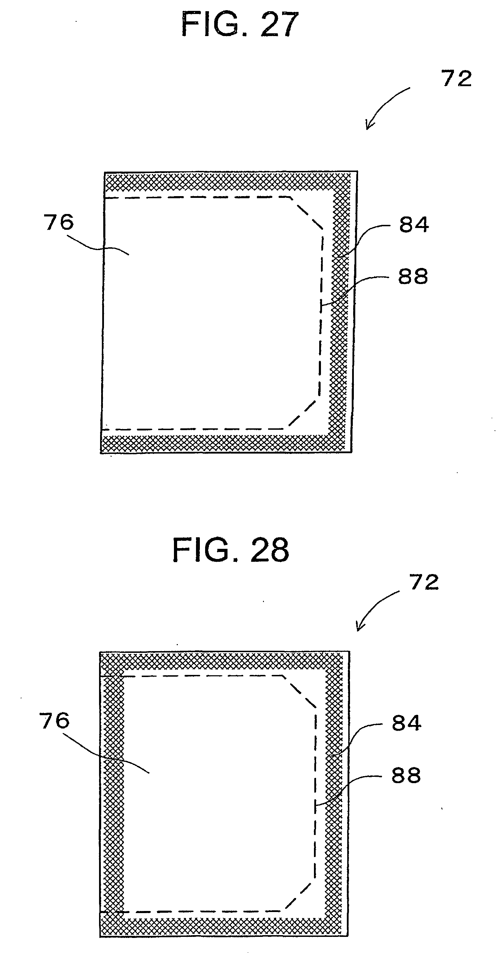

Fig. 27 is a top view of other embodiment of the wrapping body according to the invention.
Fig. 28 is a top view of further other embodiment of the wrapping body according to the invention.

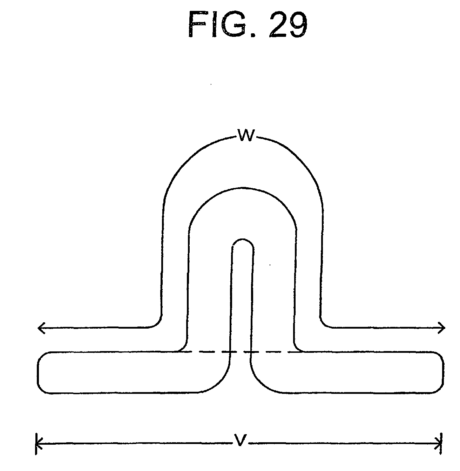

Fig. 29 is an explanation view of a size in a lateral direction of the interlabial pad.

### Best Mode of Carrying Out the Invention

Preferable embodiments of the interlabial pad in accordance with the present invention will be described with reference to accompanied drawings. Incidentally, in the specification of the present invention, the word "bulkiness" means a size of the direction which the absorbing sheet body projects, "the width" means a size of the lateral direction of the pad and "the length" means a size of the longitudinal direction of the pad.

Figs. 1(a) and 1(b) are views of an interlabial pad 14 according to the invention that Fig. 1(a) shows a top view of the pad and that Fig. 1(b) shows a cross section view along line A-A' of Fig. 1 (a).

### [(A) Structure of Basic Interlabial pad]

As shown in Figs. 1 (a) and 1(b), the basic interlabial pad 14 of the present invention comprises an absorbing sheet portion 14a and a support sheet portion 14b to back the absorbing sheet portion 14a. The absorbing sheet portion 14a includes an absorber 13 at least for absorbing the menstrual blood, the surface thereof is covered by a water permeable cover sheet 11 if desired and is used to face toward the body side in wearing it. And the absorbing sheet 14a is structured by a pair of absorbing sheet bodies 16, which are separated along the substantial centerline of the pad 14. While the support sheet 14b is structured by an impermeable support sheet 12.

The absorbing sheet body 16 and the support sheet 12 are connected with each other on the peripheral edge portion 15 of the interlabial pad 14 and the contact surface other than that is in a condition of being not connected so that a void 18 is formed between the garment face side of the absorbing sheet 16 and the body face side of the support sheet 12, through which the menstrual blood can flow into.

The whole formation of the interlabial pad 14 is eligible for attaching to the labia and is not limited to the specific one, however it is preferable to form it into a substantial oblong shape, for example, to an elliptic type an ovoid type, a gourd-shape, a drop-shape and the like. It is preferable to decide the size of the interlabial pad 14 in consideration of easily attaching to the labia. In view of them, preferably the length of the pad is 60 to 150 mm, more preferably 80 to 120 mm. Further it is preferable that the length in the lateral direction of the interlabial pad is a range of 10 to 60mm in appearance, more preferably of 20 to 40mm. In case that the length in the lateral direction is longer than 60mm, the area exceeding from the labia scrapes with the femoral region and the like of the person, and resultantly the friction which is caused by scrapping, will overcome the inching force of both labia and there is a fear of dropping off the interlabial pad. Further, in case of the length in the lateral direction of the pad being shorter than 10mm, the area of the pad in capable of interposing between labia is decreased. Therefore the contact area with labia surface is also decreased and it is easily caused a fear of dropping off the interlabial pad from labia. Incidentally, the word "in appearance" means that the length is the minimum distance between tow points (corresponding to V in Fig. 29). Since in the production process, there is a case that the distance tracing the uneven form, that is, the distance between two points in a flat condition of the pad which the uneven shape is provided is treated as a substantial distance (corresponding to W in Fig. 29), the distance is defined very carefully. In case of wrapping the absorber 13 in the cover sheet 11, to prevent the bad wear feeling caused by the peripheral edge of the absorber becoming hard, preferably the size of the absorber 13 wrapped by the cover sheet 11 is structured to be 2 to 10mm smaller than the outline of the cover sheet 11.

In the interlabial pad 14 illustrated in Fig. 1, the absorbing sheet body 16 which absorber 13 is wrapped by the cover sheet 11 is adhered on the support sheet 12 by an adhesive.

For the adhesive used for the pad, a pressure sensitive adhesive made up mainly of a synthetic rubber such as non water soluble styrene ethylene-butadiene-styrene block copolymer (SEBS), styrene-butadiene-styrene block copolymer (SBS), styrene-isoprene-styrene block copolymer (SIS) and the like, the heat sensitive adhesive made up mainly of a heat flexible resin such as ethylene-vinyl acetate copolymer (EVA) and the like, the adhesive made up mainly of water soluble heat flexible resin (such as poly vinyl alcohol (PVA)), water sensitive gel made up mainly of a starch glue, or acrylic acid and comprised of the chemical simulation, the felxibilizer or a water included therein, non water sensitive gel made up mainly of a silicone and comprised of the chemical stimulation and the flexibilizer included therein, are eligible. It may select to dispose the adhesive from a surface type, a dot type, a mesh type, a stripe type and the like.

### [Cover sheet]

For the cover sheet, the sheet-shaped material having a liquid permeable structure of a woven or non woven fabric are eligible and is not limited to the specific one. For the woven or non woven fabric material, both natural fabric and chemical fabric can be used. For example of the natural fabric, cellulose such as a grinding pulp and cotton is eligible. For example of the chemical fabric, a regenerated cellulose such as a viscose rayon and a fibril rayon, semi synthetic cellulose such as acetate and triacetate, a heat flexible hydrophobic chemical fiber which is treated in hydrophilic are eligible. For a heat flexible hydrophobic chemical fiber, a single fiber such as polyethylene (PE), polypropylene (PP), polyethylene terephthalate (PET), a fiber comprised of a graft polymerization of PE and PP, or a compound fiber of which core material is PP or PET and a myelin part is PE are eligible.

Furthermore, especially in case of using the non woven fabric, a web forming may be manufactured by a single dry method (a card method, a spun bond method, a melt blown method, a air laid method, a through air method, a point bond method and the like) or a single wet method and the like, or by a combination of plurality methods thereof. For a method of bonding, a thermal bonding, a needle punch, a chemical bonding and the like are eligible, however it is not limited to the specific methods. Further a spun lace that is formed in a sheet type by a water flow confounding may be eligible.

Among the materials, considering the liquid mobility from the inner face of the labia, chemical stimulation by an activator, and adhesion with the inner wall of the labia, it is preferable to laminate rayon with 1.1 to 4.4dtex fineness and 7 to 51 mm fiber length by 40 to 80 % of a total specific weight per unit area on the body face side, and to laminate a mixture of rayon with 1.1 to 4.4dtex fineness and 7 to 51 mm fiber length by 14 to 42 % of a total specific weight per unit area and PET with 1.1 to 4.4dtex fineness and 7 to 51 mm fiber length by 6 to 18 % of a total specific weight per unit area on the garment face side. After laminating them so that the total specific weight per unit area of the two layers becomes 20 to 60 g/m², the fibers are entangled by water - flow interlacing treatment and then dried to prepare spun lace non woven fabric with the thickness of 0.13 to 0.50 mm. The spun lace non woven prepared as described is preferable. At this time, by mixing PET on the garment face side, bulkiness can be easily maintained even if the water permeable sheet becomes wet. Therefore, adhesion to the inner wall of the labia can be maintained.

Furthermore, since for transferring the menstrual blood into the absorber, the capillarity and the water permeability is required on a part of contacting area with ostium vaginae of the cover sheet, it prefers to use a porous non woven fabric sheet having a rate of hole area from 5 to 60% and a hole area of 0.28 to 4.9mm². While in addition to the capillarity to transfer the menstrual blood into the absorber, the improved contact ability with the labia inner wall is required to the part comprising the area contacting with the labia inner wall, and it prefers to use the non woven fabric sheet, which has the surface without the unevenness and is not provided with the porous.

### [Absorber]

The absorber may be capable of keeping and absorbing a liquid (the menstrual blood) and preferably may be bulky, may not be difficult to lose the shape and may have less chemical stimulation. For the material of the absorber, cellulose (natural pulp, chemical pulp or natural cotton and the like), a regenerated cellulose (a rayon and a fibril rayon and the like), a semi synthetic cellulose (acetate and triacetate and the like), a particle water-absorbing resin, a fiber-type water-absorbing resin, a chemical fiber (a heat flexible hydrophobic chemical fiber which is treated in hydrophilic and the like) and a hydrophobic resin can be used in single or can be mixed.

The method of forming these materials to the absorber is not limited to the specific one, however, for example, the materials is manufactured into the sheet type by an air laid method, a melt blown method, a spun lace method and a paper making and the like. Concretely, the air laid pulp (or water-absorbing resin is mixed therewith), the melt blown non woven fabric treated into hydrophobic, the spun lace non woven fabric mainly comprised of hydrophobic fiber, tissue, cellulose foam, a continuous foam of the synthetic resin and the like can be used. Furthermore it is possible to grind these sheet-shaped materials and thereafter to form them into the absorber again.

It is preferable for the absorber, although any material can be used as long as it is capable of absorbing and holding liquid (body fluid), to be bulky, hard - to - be deformed, less chemically stimulant, and highly flexible to fit into the labia. Specifically, a non woven sheet in which, 50 to 150 g/m² of pulp selected from the range of the fiber length of 1 to 10 mm is laminated on the garment face side and, on the body face side, 150 to 250 g/m² of a mixture obtained by mixing 60 to 90 % of rayon with 1.1 to 4.4dtex fineness and 20 to 51 mm fiber length with 40 to 10 % of natural cotton by this mixing ratio is laminated, which then to be formed into a sheet by dotted embossing to have 2 to 10 mm bulkiness, and more preferable to have 3 to 5 mm bulkiness. Thereby, liquid can be easily transferred from the body face side to the garment face side resulting in the improvement of the absorbing and holding capacity. Furthermore, by providing a mesh spun lace non woven fabric of rayon with 1.1 to 4.4dtex fineness and 25 to 51 mm fiber length by a specific weight per unit area of 15 to 40 g/m², the liquid transferred from the body face side can be diffused by the mesh spun face to be induced to almost all over the region of the pulp layer. Therefore, more liquid can be effectively absorbed.

### [Support Sheet]

In case of using the water permeability material, the same material, which is used for the cover sheet, is eligible. In this case, it prefers to use the pad together with a sanitary napkin (a pad used together with the sanitary napkin).

Further if impermeable material is used to the support sheet, the menstrual blood, which is kept in the absorber, is prevented from a leak out of the interlabial pad. Furthermore the pad can be comprised of water vapor permeability material, thereby in wearing the pad, the sweat and the discomfort can be decreased.

To apply impermeable materials, an impermeable film such as a thin filmed synthetic resin of PE, PP and the like, a porous film comprised that a synthetic resin is filled with inorganic filler and provided with an extension treatment, a laminate film compound of a paper or non woven fabric and impermeable film, water repellent, a porous resin film is connected on a rear surface of the non woven fabric of a spun bond or a spun lace which are treated with a water repellent, are eligible. Further for a method of providing ventilation on impermeable sheet, it is eligible to form a capillary having 10 to 30% rate of the hole area and a pore size of 0.1 to 0.6 mm toward the absorber.

More concrete example of applying impermeable materials, a film mainly of a low density polyethylene (LDPE) resin is eligible which is obtained from a range of a density 0.900 to 0.925g/cm³, in amount from 15 to 30g/m² by a specific weight per unit, based on the specific weight per unit area of the composition. The flexibility not to hurt a wear feeling is considered.

Furthermore the flexibility not to hurt a fixing feeling is considered, for example, it prefers to use a film mainly of a low density polyethylene (LDPE) which is obtained from a range of a density 0.900 to 0.925g/cm³, in amount from 15 to 30g/m² by a specific weight per unit, based on the specific weight per unit area of the composition.

More preferably, during the pad is attached between labia, impermeable sheets are contacted with each other, with the pad which is used together or with the under wear, to decrease a fear of dropping the interlabial pad from the labia due to the high friction, the film is treated an embossing process and the convex upheaval portion is disposed, thereby it may decrease a ratio contact by less friction drag.

### [Mini Sheet Piece]

A material used for the mini sheet piece is selected in consideration of having an enough strength against a damage by the inserted finger, preferably it can be selected individually from or from one laminated a sheet-shaped non woven fabric, an elastic and expand non woven fabric, a film, a foam film, an elastic and expand film, a foam sheet, a tissue paper and the like.

Preferably the breaking strength in the lateral direction of the mini sheet piece, which is structured by materials described hereinbefore, is 0.1N/10mm, more preferably from 0.1 to 1.0 N/10mm. This breaking strength is a value per a width of 10mm, which is evaluated by a tensilon testing that the mini sheet piece is held with a chuck distance of 100mm and pulled by a velocity of 100mm/min.

In consideration of the above-described condition, concretely the film which has a thickness from 15 to 30µm and is mainly composed of LDPE resin having a density of 0.920g/cm³, is eligible. While for easily putting the finger in and out of the finger insertion opening of the mini sheet piece, it is preferably to select the foam film which is mainly composed of LDPE resin having a density from 0.915g/cm³ and on which a capillary which has a bulkiness from 0.3 to 1.0mm and a pore size from 0.3 to 1.5mm, is formed at the rate of hole area from 15 to 60%.

It is possible to use the material having an extensibility or an elastically expand to the lateral direction of the support sheet for inserting the finger into the mini sheet piece regardless of the finger size of the user.

For providing the extensibility to the mini sheet piece, an extensible spun bond non woven fabric may be used. The fabric has a stress from 0.1 to 0.5N/25mm in 5 % extension in case of a constant speed extension by a speed of testing of 100mm/min with a knob distance of 100mm. Further providing an elasticity and an expand for the mini sheet piece, it may be used a fiber-shaped sheet and a film sheet which a heat flexible elastomer resin is used and may be used independently an elastic and expand materials such as these heat flexible resin or a natural rubber and the like, or may be used a combination of inelastic and expand materials. Further it is a preferable embodiment to provide elasticity for a film mainly formed from LDPE resin by a corrugate treatment.

The length of the mini sheet piece is determined to hold the interlabial pad certainly and to easily put the finger into the opening. Concretely the length of the mini sheet piece prefers at least 10 % over than the length of the interlabial pad, more preferably within a range from 10 to 80%, and most preferably within a range from 30 to 60%. The width of the opening through for inserting the finger prefers at least 20mm or more, more preferably within a range from 20 to 50mm, and most preferably within a range from 30 to 40mm.

With respect to the form of the mini sheet piece, for example, as shown in Fig. 19, it is eligible to form that along the garment face side of the support sheet 42 comprising the interlabial pad 44, the strip-shaped mini sheet piece 52 is horizontally disposed in the lateral direction of the interlabial pad 44. In this example, the mini sheet piece 62 is fixed at both side ends of the interlabial pad 44 and the finger insertion opening 64, that is the opening for inserting the finger, is formed toward the longitudinal direction of the interlabial pad 44.

In the embodiment, when the finger 78 is inserted into the finger insertion opening 64 with contacting a ball of a finger 78 to the support sheet 42, as illustrated in Fig. 20, the longitudinal direction of the interlabial pad 44 and a direction of pudendal slit 80, face in the same direction. Therefore the interlabial pad 44 can be push into the inside of the labia by a ball of the finger 78 so as to attach the interlabial pad 44 certainly.

Further the mini sheet piece may be formed as illustrated in Fig. 21, for example, that the support sheet 42 which comprises the interlabial pad 44, is completely covered from the near central portion in the longitudinal direction to an edge end 82 in the longitudinal direction of the interlabial pad 44. The embodiment described hereinbefore is preferable for a sanitary handling of the pad so that the finger end 78 is prevented from exposing out of the mini sheet piece 62 and non-bonding condition of the menstrual blood and the finger 78 can be maintained.

Incidentally, for example, as illustrated in Fig. 22, the end of the finger 78 is also prevented from exposing out of the mini sheet piece 62 by the interlabial pad 44 which a plurality of strip-shaped mini sheet pieces 62 are formed at distance, thereby the effect of the sanitary handling can be achieved as same as the interlabial pad 44 as shown in Fig. 20.

Fig. 23 is a top view of the interlabial pad 44, and Fig. 24 is a section view along line A-A' of the interlabial pad of Fig. 23. In case of providing the mini sheet piece, for example, as illustrated in Fig. 24, it prefers to form the void 83 to insert the finger preliminarily by somewhat rising the mini sheet piece 62 from garment face side of the support sheet 42.

In this embodiment, the width of the mini sheet piece 62 is formed somewhat larger than that of the interlabial pad 44 and the piece 62 is bonded with the periphery edge portion 45 of the interlabial pad 44 in somewhat loose condition not to contact with the garment face side of the support sheet 42. The method of fixing the pad is not limited specifically, for example, the method of pressure sensitive hot melt adhesive, heat sensitive hot melt adhesive, heat seal, ultra sonic seal and the like will be eligible. The application of the adhesive can be selected from, for example, a surface-shaped, a line-shaped, a spiral-shaped, a dot-shaped and the like.

In the interlabial pad 44, as illustrated in Fig. 24, the side end of the mini sheet piece 62 is bonded with the periphery edge portion 45. In this embodiment, to prevent the periphery edge portion 45 from the stiff wear feeling of the interlabial pad 44, preferably the width of bonding portion is set in a range from 2 to 5 mm. Further the mini sheet piece 62 is bonded inwardly (toward the side of the central portion) of the interlabial pad 44 from the periphery edge portion 45, thereby preferably a soft feel of materials is provided on the periphery edge so as to increase a good wear feeling. In this embodiment, it is preferred that the mini sheet piece 62 is adhered on the support sheet 42 by hot melt-typed adhesive.

Further, for bonding between the mini sheet piece and the support sheet with an adequate strength, in respect of the bonding strength, it is preferably to set the breaking strength in a lateral direction of the pad in a range from 0.3 to 1.2 N/10mm. Incidentally, this breaking strength is a strength per a width of 10mm and a value which is evaluated by a tensilon testing that the mini sheet piece held on the upper chuck and the support sheet held on the lower chuck with a chuck distance of 20mm,are pulled by a velocity of 100mm/min.

Furthermore, to achieve an easy identification of the mini sheet piece by the user, the mini sheet piece can be adjusted to have a different color or design, chromaticity from the support sheet by coloring or printing patterns.

### [Wrapping Container]

The conventional wrapping container can be used for wrapping the interlabial pad according to the invention. For example, non woven fabric comprised of PE, PP, PET and the like, a film having a thickness from 15 to 60µm, a paper or laminate materials treated by a lamination of these materials is eligible.

Incidentally, in consideration of a soft feeling, the inner face side of the wrapping container is preferably comprised of materials so that is a crepe tissue in a range from 15 to 50g/m² by specific weight per unit area, wet spun lace non-woven fabric in amount from 15 to 70g/m² by specific weight of a composition of a cotton and a pulp and including a cotton in amount of 10% by mass at least, a spun lace non-woven fabric in a range from 20 to 70g/m² based on the specific weight of the composition and including a rayon in amount of 30% by mass or more, or melt blown non-woven fabric comprised of PP in amount from 20 to 50g/m² by specific weight per unit area. Further it is also preferable to comprise the container by a composed non-woven fabric which is comprised a melt blown non-woven fabric in a range from 5 to 20g/m² by a specific weight per unit is held between the spun bond non-woven fabric in amount from 6 to 10g/m² by specific weight per unit area. While it is preferable to comprise the outer surface side of the wrapping container in consideration of water durable pressure by a film comprising PE in amount from 10 to 30g/m² by specific weight per unit area or a porous plastic sheet having a rate of hole area from 10 to 30% and in amount from 15 to 30g/m² by specific weight per unit area.

The inner and outer face side materials of the wrapping container are laminated in unit by a conventional method of a hot melt adhesive, heat emboss ing or an ultra sonic sealing and the like. In this embodiment, it is preferable to apply the hot melt adhesive in a spiral-shaped or a line-shaped with application coverage from 3 to 10g/m² and with a rate of applicant area from 5 to 40%. In case of a heat embossing or an ultra sonic sealing, the adhesive is applied in a line-shape, a dot-shape or a cross line-shape and the like with a rate of a sealing area from 5 to 20% in consideration of the feeling of the laminate material.

### [Wrapping body]

The wrapping body in accordance with the present invention wherein the interlabial pad is contained in a wrapping container includes, other than the aforementioned wrapping bodies, for example, the embodiment illustrated in Figs, 25 and 26. In the wrapping body 72, the interlabial pad 44 is folded so that the mini sheet piece 62 faces along the substantial center line of the longitudinal direction, the interlabial pad 44 is wrapped in one wrapping sheet subject to be a wrapping container 76 as illustrated and the peripheral end portion of the sheet is sealed. In the embodiment, two wrapping sheets may hold the interlabial pad 44 and the edge portion of the sheet is sealed. Incidentally, the mini sheet piece 62 is folded in facing along the centerline, thereby the user can confirm the mini sheet piece 62 by eyes in opening the wrapping container 72.

In the wrapping container 76 illustrated in Fig. 25, 26, the portion 84 is sealed with a condition in capable of breaking and more preferably for easily opening and for preventing the pad from a damage in using, that the portion is sealed by a heat seal with the breaking strength in a range from 0.3 to 1.0N/25mm, which is evaluated testing with a velocity of 100mm/min by a tensilon testing. In the wrapping container illustrated in Fig. 25 and Fig. 26, the knob 86 is provided and the wrapping container 76 can be broken by picking two knobs 86.

In comprising the wrapping container by sealing the wrapping sheet, other than the wrapping container 76 as illustrated in Figs. 25, 26 which is opened by breaking the portion 84, as illustrated in Fig. 27 and Fig. 28, it may be possible to structure that the container is opened by breaking the perforation 88 to be formed on the inner peripheral side of the portion 84. In the wrapping body 72 illustrated in Fig. 27, the interlabial pad is folded simultaneously with the wrapping sheet so as to face rear surface sides with each other, thereafter three edge portions of the wrapping body are sealed by a heat seal and the perforation is formed on the inner peripheral side. In the wrapping body 72 illustrated in Fig. 28, the pad is folded in a manner that the rear surface sides face with each other, thereafter is wrapped by two wrapping sheets, four edge portions are sealed by the heat seal and the perforation is formed on the inner peripheral side.

In consideration of preventing the pad from damage in keeping it and of easily breaking the wrapping in using, the breaking strength of the perforation 88 is preferably in a range from 0.2 to 3.0N/25mm, further from 0.3 to1.5N/25mm. Further for easily breaking the perforation, it is required to start the perforation 88 from the end of the wrapping container 76 as illustrated, while the perforation 88 may be formed continuously or intermittently if it can be broken. Furthermore in case of forming the perforation 88, it is also preferable that the strength on the portion 84 is increased, since it is prevented from misunderstanding to break and open such as the bonding portion 84 of the wrapping container 76 as illustrated in Figs. 25, 26.

For any embodiment illustrated in Fig. 27 and Fig. 28, it prefers that the user can easily confirm the start point of the perforation by printing an arrow confirmed by eyes, or by cutting around the start point of the perforation in a different formation from other portions or by forming whole wrapping body in unsymmetrical design.

### [(B) Structure of the interlabial pad provided with biodegradability, water dispersibility and water solubility]

Preferably the interlabial pad is comprised of a material of biodegradability and/or a material of water dispersibility and/or a material of water solubility. After using the pad comprised of these materials, it can be disposed into a toilet to flush, thereby the destruction of the pad can be easily and sanitarily achieved and the garbage in a toilet can be decreased.

In this Specification, "biodegradability" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment. The description "water dispersibility" has a same means of water solubility and that is not effected from restricted volume of fluid (menstrual blood), however in a volume of water or water flow, fiber themselves are easily dispersed into small piece to the extent not to damage the normal toilet pipe. "Water solubility" means a nature that is not affected by restricted volume of fluid (menstrual blood) in using, however is dissolved by a volume of water or water flow.

### [Cover Sheet]

Any of natural fiber and chemical fiber can be used for materials of the cover sheet to achieve biodegradability, water dispersibility and water solubility. For example of natural fiber, there are cellulose such as a grinding pulp and cotton and air laid pulp and the like which is chemically composed by water-soluble resin. For examples of chemical fiber, there are regenerated cellulose such as rayon, fibril rayon, PE, PP, PET, materials which is hydrophilic treated to the chemical fiber such as ethylene-vinyl acetate copolymer, moreover poly lactic acid so-called biodegradable fiber, and polybutylene succinate and the like. Further water-soluble carboxymethylcellulose and polyvinyl alcohol are eligible for use. In these materials, it prefers to use biodegradable fiber that are cellulose such as a pulp and cotton, regenerated cellulose such as rayon and the like and poly lactic acid.

Incidentally, materials described hereinbefore can be used independently or be mixed by forming a web or the non-woven fabric. For web forming biodegradable fabric such as poly lactic acid or polybutylene succinate and the like, any of a card method, a spun bond method, a melt blown method or a dry method or a wet method by an air laid method, or a combination of these is eligible. For a method of bonding, a thermal bonding, a needle punch bonding, a chemical bonding and the like are eligible. However, it is not limited to these method. Further, a spun lace formed in a sheet-shaped by a water flow confounding is eligible.

For an example of a forming method of applying water dispersibility, a method of producing a water soluble paper which the fiber is formed in the sheet-shaped by a hydrogen bonding of fiber themselves, a method of producing water soluble paper which fibers are bonded each other into sheet-shaped by water soluble binder or a method of producing water soluble paper which fibers are confounded into sheet-shaped are eligible.

Incidentally, preferably a length of fiber in a range from 2 to 51 mm, most preferably in a range from 2 to 10mm can achieve a good water dispersibility. Further in addition to water dispersibility, preferably fineness (thickness) of fibers is selected from a range of 1.1 to 4.4 dtex for also having strength not to damage the pad in use. Especially in using rayon as a fiber, it prefers to select the fiber having fineness from 1.1 to 3.3 dtex. In case of fineness being less than the value described above, it is possible to achieve water dispersibility, however in a dry condition, water dispersibility is extremely deteriorated by easily becoming fuzz or coming out of fuzz from fibers.

Preferably the amount of the cover sheet is selected from 20 to 60g/m² by a specific weight per unit based on specific weight per unit area. Further it is required that the breaking strength of the cover sheet is 800mN/25mm at least in both vertical and horizontal directions and preferably is from 1000 to 7000mN/25mm in consideration of the softness in wearing the pad (the breaking strength of the sheet evaluated from the constant speed extension by a speed testing 100mm/min in a condition of a knob distance 100mm).

For further concrete structure of the cover sheet, a wet spun lace non-woven fabric is eligible, that a rayon having a fineness of 1.1 to 4.4dtex and a length from 5 to 10mm and a wood pulp are mixed in a ratio from 90: 10 to 70: 30 by mass to adjust the amount from 25 to 40g/m², and the thickness from 0.2 to 0,5mm. In order to substantially improve the water permeability of the menstrual blood or to apply an good image of the menstrual blood permeability, a plurality of holes can be also provided on the sheet in configuration having a hole diameter from 0.5 to 1.5mm and a hole area ratio (a ratio of opening hole in respect of whole area) from 3 to 20%.

### [Absorber]

Same materials having water permeability for the cover sheet can be used for materials of the absorber to apply biodegradability, water dispersibility and water solubility. Further it is possible to independently use the absorber such as alginic acid soda, starch, carboxymethylcelluloce and the like, particle-shaped or fiber-shaped high absorbing polymer, or to use a form by mixing these materials with same materials for the cover sheet.

In respect of the specific structure of the absorber, for example, the wood pulp and the like are eligible, that is laminated to the amount from 150 to 500g/m² by a specific weight per unit to enclose into tissue and is adjusted the thickness from 2 to 10mm by a press device. It is possible to improve the absorption capacity or keeping ability of the menstrual blood by mixing absorber such as starch and the like in a ratio from 5 to 30g/m².

### [Support Sheet]

For materials of the support sheet having biodegradability, water dispersibility and water solubility, and further impermeability cellulose guide materials such as methyl cellulose, hydroxyethylcellulose , carboxymethylcellulose and the like, water solubility polymer such as polyvinyl alcohol, alginic acid, polyacrylic acid soda, polyacrylic ether, polyvinylpyrrolidone, a copolymer of isobutylene and dry maleic acid , or biodegradable polymer such as poly lactic acid, polybutylene succinate, starch and dextrin are eligible.

These materials can be formed in the film-shaped or the sheet-shaped in individual or by a mixture. At least on one surface, preferably on both surfaces of the film sheet, waterproof materials such as silicone resin can be applied or mixed. The laminate paper comprised of the non-woven fabric which is treated by laminating tissue, is eligible. Further if necessary, the support sheet can be applied the coloring by mixing the mineral pigment in amount from 0.1 to 5 % of mass.

In respect of the specific structure of an impermeable support sheet, for example, a laminate paper is formed by a laminating treatment that a film and tissue are bonded. The film comprises poly lactic acid and tissue has a thickness from 10 to 20 µm, in the amount from 15 to 20g/m² by a specific weight per unit based on the specific weight per unit area, are laminated in area ratio from 5 to 40%. The laminate paper described hereinbefore can keep impermeability during the pad is wet and it is preferable in preventing the digestion tank from an exceed damage.

### [Mini Sheet Piece]

In respect of materials for the mini sheet piece to apply biodegradability, water dispersibility and water solubility, poly lactic acid, polybutylene succinate, a film comprised from PVA and the like, or materials laminated the film of these materials with tissue are eligible.

### [Wrapping Container]

For applying biodegradability, water dispersibility and water solubility ability, the container may be comprised of a fiber sheet using water soluble fiber, a film using biodegradable resin or water soluble resin, or a laminated materials of the fiber sheet with the film, a laminated materials of the film and tissue.

### [Bonding Method]

Further for a bonding method of applying biodegradability, water dispersibility, water solubility, a bonding method such as adhesion by polyvinyl alcohol and the like having water solubility or water swelling, a heat sealing, or a bonding by a hydrogen bonding, and the like can be used individually or can be used in a combination of them adequately.

### Industrial Applicability

As described hereinbefore, in the present invention, the absorbing sheet is comprised of a pair of absorbers which are divided along the substantial center line of the interlabial pad, the absorbing sheet and the impermeable support sheet are connected with each other on the periphery edge portion of the interlabial pad to form the void between the absorbing sheet and the support sheet through which the menstrual blood can flow into, thereby the menstrual blood flows along the inner wall of the labia and can be rapidly transmitted into the absorber. Further the fitting ability of the pad in the labia can be improved.

## Claims

1. An interlabial pad for attaching to labia, comprising:
an absorber for absorbing body fluid;
an absorbing sheet portion facing to a body side upon wearing the interlabial pad; and
a support sheet portion backing the absorbing sheet portion;
wherein said absorbing sheet portion comprises a pair of absorbing sheet bodies which are separated along a substantial center line of the interlabial pad, and
said absorbing sheet body is bonded to said support sheet portion comprising an impermeable support sheet on a peripheral edge portion of the interlabial pad, thereby the body fluid can flow into a void which is formed between a garment face side of the absorbing sheet body and the body face side of the support sheet.

2. An interlabial pad according to claim 1, wherein a side end of the interlabial pad has a shape of an arc, and both peripheral ends of the arc converge toward edge ends of the substantial center line along a longitudinal direction of the interlabial pad.

3. An interlabial pad according to claim 1 or 2, wherein said pair of absorbing sheet bodies are separated each other, and a groove portion is provided between the absorbing sheet bodies.

4. An interlabial pad according to any one of claims 1 to 3, wherein on a body face side of the support sheet, an strip-shaped absorber is disposed along a dividing line of a pair of absorbing sheet bodies.

5. An interlabial pad according to any one of claims 1 to 4, further comprising a sheet-shaped absorber being disposed on a body face side of the support sheet, wherein a void is formed between the garment face side of the absorbing sheet body and the body face side of the sheet-shaped absorber whereby body fluid can flow into said void.

6. An interlabial pad according to any one of claims 1 to 5, further comprising
a mini sheet piece, in the garment face side of the support sheet, including:
one or more bonding portions on each side portion in respect of a longitudinal direction of the support sheet; and
one or more disconnecting portion in a lateral direction of the support sheet,
wherein at least one of said one or more disconnecting portion form an opening for receiving a finger between the mini sheet piece and the support sheet so as to directly keep the opening having a finger breadth in a surface direction of the support sheet.

7. An interlabial pad according to claim 6, wherein at least a pair of absorbing sheet bodies include:
an inclining portion having a mountain shape in a body side direction, the mountain shape having a vertex in the substantial center line along the longitudinal direction of the interlabial pad and a pair of side ends; and
a hem portion continuing from both of the side ends of said inclining portion,
wherein the mini sheet piece is bonded on the garment side of said hem portion.

8. An interlabial pad according to any one of claims 1 to 7, wherein the interlabial pad is used together with a sanitary napkin.

9. An interlabial pad according to any one of claims 1 to 8, wherein said interlabial pad is a pad for incontinence of urine.

10. An interlabial pad according to any one of claims 1 to 8, wherein said interlabial pad is a pad for absorbing vaginal discharge.

11. A wrapping body comprising:
the interlabial pad according to claims 1 to 10; and
a wrapping container for wrapping the interlabial pad individually.

12. A wrapping body comprising:
the interlabial pad according to any one of claims 6 to 10; and
a wrapping container for wrapping the interlabial pad individually, said wrapping container having an unwrapped opening,
wherein said interlabial pad is so contained in said wrapping container that said finger insertion opening faces and opens toward the unwrapped opening of the wrapping container.

13. A wrapping body according to claim 12 wherein the interlabial pad is contained in the wrapping container such that the mini sheet piece is mountain-folded toward garment side directions along the substantial center line in the longitudinal direction of the interlabial pad.
